# EUROPÄISCHE PATENTANMELDUNG

(11) **EP 0 905 513 A2**
(43) Veröffentlichungstag der Anmeldung: **31.03.1999**
(21) Anmeldenummer: 98116425.4
(22) Anmeldetag: 31.08.1998
(51) Int. Cl.: G01N 33/36, G01J 5/00

(54) **Verfahren zur Erkennung des Feuchtegrades**

(30) Priorität: 01.09.1997 DE 19738150
(71) Anmelder: Mahlo GmbH & Co. KG, 93342 Saal (DE)
(72) Erfinder: Epple, Helmut, Ing., 93342 Saal/Donau (DE); Daul, Robert, Dipl.-Ing., 86316 Friedberg-Wiffertshausen (DE)
(74) Vertreter: Bohnenberger, Johannes, Dr.

(57) **Zusammenfassung**

Die Erfindung betrifft ein Verfahren zur Erkennung des Feuchtegrades einer vorzugsweise textilen Warenbahn. Dabei durchläuft die Warenbahn eine Feuchteerfassung. Zur Ermittlung des Absolutwertes des Feuchtegrades erfolgt eine erste Feuchtemessung und zur Erfassung des Feuchteprofils quer zur Warenbahn eine zweite Feuchtemessung über einen vorgegebenen Erfassungsbereich.

## Beschreibung

Die Erfindung betrifft ein Verfahren zur Erkennung des Feuchtegrades einer Vorzugsweise textilen Warenbahn sowie eine entsprechende Vorrichtung.

Zur Überwachung und Regelung der gleichmäßigen Trocknung einer vorzugsweise textilen Warenbahn wird ein Verfahren bzw. eine Vorrichtung zur Feuchtemessung benötigt. Es ist bereits eine Vorrichtung bekannt, bei der die Restfeuchte der Warenbahn am Auslauf des Trockners mit drei Paaren von Meßrollen abgetastet wird. Die Widerstands-Meßrollen sind gleichmäßig beabstandet über die Breite der Warenbahn verteilt angeordnet. Die Warenbahn läuft jeweils zwischen den Rollen eines Paares hindurch, so daß der Widerstand der feuchten warenbahn zwischen den zugeordneten Meßrollen, also punktuell an drei Stellen, ermittelt werden kann.

Dabei tritt aber das Problem auf, daß die Widerstands-Meßrollen auf empfindlichen warenbahnen Spuren hinterlassen können. Warenbahnen aus empfindlichem Textilgut können daher nicht zwischen beabstandeten Meßrollen hindurchgezogen werden. Vielmehr ist in einem solchen Fall ein Paar von Meßwalzen vorgesehen, deren Breite die Breite der Warenbahn überdeckt. Die Warenbahn wird dann zwischen den beiden Meßwalzen des Walzenpaares hindurchgeführt. Mit den Meßwalzen läßt sich aber nur ein Feuchtemaximalwert detektieren. Der ermittelte Widerstand wird nämlich weitestgehend durch die feuchteste Stelle auf der Warenbahn bestimmt.

Es kommt immer wieder vor, daß die Warenbahn ein ungleichmäßiges Trocknungsprofil aufweist. Beispielsweise kann durch Unausgewogenheiten der Trocknerleistung des Spannrahmens eine ungleichmäßige Trocknung erfolgen. Selbst wenn die Warenbahn an sich einen gleichmäßigen Trocknungsvorgang durchlaufen hat, können auf der Warenbahn ein oder mehrere Feuchtigkeitsstreifen vorhanden sein. Die Ursache für solche Feuchtigkeitsstreifen kann beispielsweise eine Foulard-Walze sein, die, etwa bedingt durch einen unsanften Kontakt mit Metallteilen, Scharten aufweist und deshalb über die Breite der Warenbahn betrachtet nicht mehr gleichmäßig abquetscht. Dort, wo der Abquetschdruck geringer ist, bleibt die Ware feuchter und läuft damit auch feuchter in nachfolgende Trocknerstufen ein.

Im Ergebnis können sich auf einer Warenbahn ein oder mehrere Feuchtigkeitsstreifen oder allgemeiner feuchte Bereiche befinden, die bei der Restfeuchtemessung nicht erfaßt werden oder insgesamt eine zu hohe Feuchtigkeit vortäuschen. Bei Verwendung von Meßwalzen wird als Feuchtegrad immer die Feuchte des feuchtesten Bereichs erfaßt. Ist nur ein lokaler feuchter Streifen vorhanden, die Warenbahn ansonsten aber relativ trocken, werden anschließende Bearbeitungsvorgänge, beispielsweise weiteres Trocknen, falsch gesteuert. Meßtechnisch gesehen wirkt ein Feuchtigkeitsstreifen auf der Warenbahn so ähnlich wie ein lokaler "Kurzschluß". Die erfaßte Feuchtigkeit entspricht weitestgehend der des Feuchtigkeitsstreifens und nicht der der restlichen Warenbahn.

Bei der Messung mit Meßrollen können die Rollen beispielsweise einen besonders feuchten oder einen besonders trockenen Bereich überstreichen, was in beiden Fällen die Messung verfälscht. Überstreichen die Meßrollen einen besonders feuchten Bereich, wird die Warenbahn als wesentlich feuchter angenommen als ihrem tatsächlichen Feuchtegrad entspricht. Bei einer nachfolgenden Trocknung wird die Warenbahn übertrocknet.

Laufen die Meßrollen gerade auf relativ trockenen Bereichen, wird die Warenbahn als bereits sehr getrocknet angenommen.

Nachfolgende Trocknungsprozesse werden dann nicht mit der nötigen Intensität durchgeführt und die Warenbahn bleibt letztendlich zu feucht.

Die Aufgabe der vorliegenden Erfindung besteht darin, ein Verfahren zur Erkennung des Feuchtegrades einer vorzugsweise textilen Warenbahn und eine entsprechende Vorrichtung anzugeben, bei der die tatsächlich Feuchte der Warenbahn, insbesondere ein durchschnittlicher Feuchtegrad über die Breite der Warenbahn verläßlicher bestimmt werden kann.

Diese Aufgabe wird in verfahrenstechnischer Hinsicht durch die Merkmale des Anspruches 1 und in vorrichtungstechnischer Hinsicht durch die Merkmale des Anspruches 9 gelöst. Vorteilhafte Weiterbildungen sind in den Unteransprüchen angegeben.

Ein Kerngedanke der vorliegenden Erfindung besteht darin, eine erste Feuchtemesssung zur Erfassung eines Absolutwertes des Feuchtegrades mit einer zweiten Feuchtemessung, die ein Feuchteprofil, insbesondere ein Feuchteprofil quer zur Warenbahn liefert, zu kombinieren. Vorzugsweise wird das in der unabhängigen zweiten Feuchtemessung erhaltene Feuchteprofil mit dem in der ersten Feuchtemessung gewonnenen Absolutwert des Feuchtegrades geeicht.

Bevorzugtermaßen erfolgt die zweite Feuchtemessung kontaktlos. Eine kontaktlose Erfassung hat den Vorteil, daß auch eine Warenbahn aus empfindlichem Textilgut durch die Abtastung nicht beeinträchtigt wird. Weiterhin arbeitet eine kontaktlose Erfassung annähernd verschleißfrei. Das Meßergebnis wird durch den kontaktlosen Abtastvorgang praktisch nicht beeinflußt.

Eine besondere Idee der vorliegenden Erfindung liegt darin, das Feuchteprofil der Warenbahn über eine Temperaturmessung zu erfassen. Dieser Vorgehensweise liegt die Erkenntnis zugrunde, daß die Temperatur der Warenbahn abhängig ist von ihrer Feuchtigkeit. Aus einem erfaßten Temperaturprofil läßt sich daher auf das Feuchteprofil der Warenbahn schließen.

Das Temperaturprofil quer zur Warenbahn und damit das Feuchteprofil lassen sich vorzugsweise in einer pyrometrischen Abtastung, d.h. unter Ausnutzung der von der Warenbahn ausgesandten Temperaturstrahlung ermitteln.

Das Temperaturprofil aus der pyrometrischen Abtastung liefert eine genaue Lagezuordnung der Feuchtigkeitsverteilung quer zur Warenbahn. Bereiche unterschiedlicher Feuchte, zum Beispiel ein Feuchtigkeitsstreifen, können genau lokalisiert werden. Die dargestellten Unterschiede im Temperaturprofil geben dabei ein relatives Maß für die Gleichmäßigkeit der Trocknung wieder. Die erste Feuchtemessung, die vorzugsweise über eine Abtastung des Widerstandes erfolgt, liefert dazu als Bezugspunkt den absoluten Feuchtewert an der feuchtesten Stelle.

Die erste und zweite Feuchtemessung können vorzugsweise zeitlich und räumlich versetzt erfolgen, derart, daß die Meßergebnisse für eine bestimmte Längsposition auf der Warenbahn, die zeitversetzt erfaßt werden, im Nachhinein verknüpft werden können. Durchläuft ein bestimmter Längsabschnitt der Warenbahn zuerst die zweite Feuchtemessung und je nach Transportgeschwindigkeit der Warenbahn eine bestimmte Zeitspanne T₁ später die erste Feuchtemessung, so werden die Abtastergebnisse der zweiten Feuchtemessung um die Zeitspanne T₁ zurückgehalten und mit entsprechender Verzögerung mit den Abtastwerten der ersten Feuchtemessung verknüpft.

Prinzipiell ist es aber auch denkbar, die erste Feuchtemessung in Laufrichtung der Warenbahn vor der zweiten Feuchtemessung anzuordnen. In dem Fall müssen die Abtastergebnisse der ersten Feuchtemessung um eine sich aus der Transportgeschwindigkeit der Warenbahn und dem Abstand der Abtasteinrichtung ergebenden Zeitspanne verzögert werden.

Un das Feuchteprofil quer zur Warenbahn zu erfassen, ist das Pyrometer parallel zu den Flachseiten der Warenbahn schwenkbar angeordnet. Es ist aber auch denkbar, in einem einzigen Abtastvorgang ein komplettes Strahlungsbild über die gesamte Breite und ggf. zusätzlich über eine gewisse Länge der Warenbahn aufzunehmen und daraus das Feuchteprofil abzuleiten.

Die Erfindung wird nachstehend auch hinsichtlich weiterer Merkmale und Vorteile anhand der Beschreibung eines Ausführungsbeispiels und unter Bezugnahme auf die beiliegenden Zeichnungen näher erläutert.

Hierbei zeigen:
- Fig. 1: eine Prinzipskizze einer Ausführungsform einer erfindungsgemäßen Vorrichtung zur Erkennung des Feuchtegrades einer vorzugsweise textilen Warenbahn,
- Fig. 2: eine Prinzipskizze einer Warenbahn mit Feuchtigkeitsstreifen.

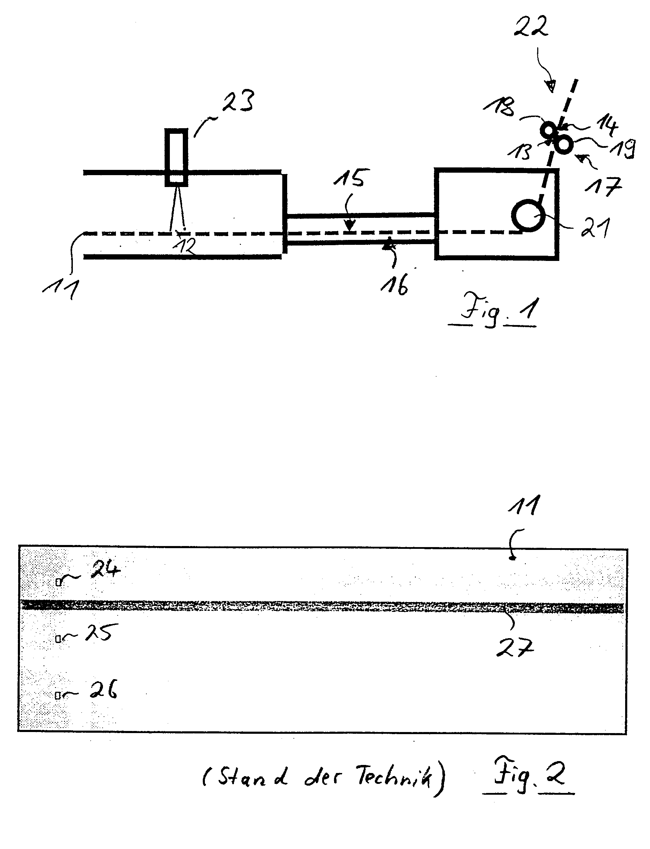

In Fig. 2 ist ein Längsausschnitt aus einer Warenbahn 11 in Draufsicht schematisch dargestellt. Die in Fig. 2 dargestellte Warenbahn 11 wird an drei Rollenpositionen, nämlich einer ersten Rollenposition 24, einer zweiten Rollenposition 25 und einer dritten Rollenposition 26 auf ihren Feuchtegrad mittels einer elektrischen Widerstandsmessung abgetastet. Ein parallel zu den Längsrändern der Warenbahn 11, jedoch zwischen den Rollenpositionen 24, 25 und 26 verlaufender Feuchtestreifen 27 wird nicht erfaßt. An den Rollenpositionen 24, 25 und 26 wird damit ein Feuchtegrad erfaßt, der vom tatsächlichen mittleren Feuchtegrad der Warenbahn über ihre gesamte Breite verschieden sein kann.

Diese Nachteile lassen sich mit einem erfindungsgemäßen Verfahren bzw. einer erfindungsgemäßen Vorrichtung beseitigen. Eine Ausführungsform einer solchen Vorrichtung ist in Fig. 1 in einer Prinzipskizze dargestellt. Die Warenbahn 11 ist strichliert von der Seite gesehen dargestellt. In der Vorrichtung ist eine erste Abtasteinrichtung 22 und eine zweite Abtasteinrichtung 23 an verschiedenen Längspositionen der Warenbahn angeordnet. Die Warenbahn 11 wird in einer Führungs- oder Transporteinrichtung 21 geführt, die hier zwischen der ersten Abtasteinrichtung 22 und der zweiten Abtasteinrichtung 23 eine Umlenkrolle umfaßt. In der ersten Abtasteinrichtung 22 wird der Feuchtegrad der Warenbahn durch eine elektrische Widerstandsmessung ermittelt. Zu diesem Zweck wird die Warenbahn zwischen einem Walzenpaar 17 mit einer ersten Walze 18 und einer zweiten Walze 19 hindurchgeführt. Die jeweiligen Anlagepunkte bzw. - linien definieren eine erste und eine zweite Meßstelle 13, 14.

Über ein zwischen der ersten Walze 18 und der zweiten Walze 19 anliegendes Potential und dem tatsächlich fließenden Strom wird mittels dieser Widerstandsmessung der Feuchtegrad der Warenbahn über die gesamte Breite ermittelt. Sollte die warenbahn Bereiche oder Streifen höherer Feuchtigkeit aufweisen, so wird der Stromfluß zwischen den Walzen 18 und 19 gerade an diesen feuchten Bereichen oder Streifen erfolgen.

In jedem Fall kann mit der ersten Abtasteinrichtung 22 ein Absolutwert für den Feuchtegrad der Warenbahn - entweder über die gesamte Breite oder wenigstens für die feuchtesten Stellen - ermittelt werden.

Ob die Warenbahn 11 über ihre Breite ein gleichmäßiges Feuchteprofil oder Bereiche höherer Feuchte aufweist, kann in einer zweiten Abtasteinrichtung 23 ermittelt werden. Die zweite Abtasteinrichtung 23, die hier als Pyrometer ausgebildet ist, ist parallel zur ersten oder zweiten Flachseite 15 oder 16 der Warenbahn 11 verschwenkbar angeordnet. Über einen Erfassungsbereich 12 erfaßt sie im wesentlichen in einer Ausrichtung von 90° relativ zur Flachseite 15 der Warenbahn 11 die von der Warenbahn 11 ausgesandte Temperaturstrahlung.

Dieser Vorgehensweise liegt die Erkenntnis zugrunde, daß der Feuchtegrad der Warenbahn bzw. das Feuchteprofil der Warenbahn direkt mit dem Temperaturprofil der Warenbahn korreliert ist.

Aus dem Temperaturprofil läßt sich daher unmittelbar das Feuchteprofil gewinnen.

Dadurch, daß die zweite Abtasteinrichtung, die hier als Pyrometer ausgebildet ist, das Feuchteprofil quer über die Warenbahn erkennen kann, also über die Breite der Warenbahn die Feuchteverteilung feststellt, kann insbesondere durch Kombination mit der ersten Abtasteinrichtung 22 die tatsächliche Feuchte und der Feuchtegrad der Warenbahn ermittelt und warenlängenbezogen protokolliert werden. Da die erste Abtasteinrichtung 22 und die zweite Abtasteinrichtung 23 warenlängenbezogen versetzt angeordnet sind, müssen die Daten der einen Abtasteinrichtung um eine Zeitkonstante, die sich aus der Transportgeschwindigkeit der Warenbahn und dem Abstand der Abtasteinrichtungen ergibt, verzögert werden, um die Daten für einen Warenlängenbereich sinnvoll zu korrelieren.

Der weitergehende Verarbeitungsprozeß oder eventuell der vorausgegangene Verarbeitungsprozeß lassen sich über die nun mögliche, wesentlich verbesserte Erfassung des Feuchtegrades und des Feuchteprofils der Warenbahn wesentlich genauer steuern.

### Bezugszeichenliste

- 11: Warenbahn
- 12: Erfassungsbereich
- 13: erste Meßstelle
- 14: zweite Meßstelle
- 15: erste Flachseite
- 16: zweite Flachseite
- 17: Walzenpaar
- 18: Walze (erste)
- 19: Walze (zweite)
- 21: Führungs- oder Transporteinrichtung
- 22: erste Abtasteinrichtung
- 23: zweite Abtasteinrichtung, Pyrometer
- 24: erste Rollenposition
- 25: zweite Rollenposition
- 26: dritte Rollenposition
- 27: Feuchtestreifen

## Patentansprüche

1. Verfahren zur Erkennung des Feuchtegrades einer vorzugsweise textilen Warenbahn (11),
- wobei die Warenbahn (11) eine Feuchteerfassung durchläuft,
**dadurch gekennzeichnet,**
- daß zur Ermittlung des Absolutwertes des Feuchtegrades eine erste Feuchtemessung, und
- daß zur Erfassung des Feuchteprofils insbesondere quer zur Warenbahn (11) eine zweite Feuchtemessung über einen vorgegebenen Erfassungsbereich (12) durchgeführt wird.

2. Verfahren nach Anspruch 1,
**dadurch gekennzeichnet,**
daß die zweite Feuchtemessung über eine kontaktlose Abtastung, vorzugsweise über eine von der Warenbahn emittierte Temperaturstrahlung (pyrometrische Abtastung) erfolgt.

3. Verfahren nach Anspruch 1 oder 2,
**dadurch gekennzeichnet,**
daß die zweite Feuchtemessung, insbesondere die pyrometrische Abtastung, in einer Schwenkbewegung parallel zu den Flachseiten (15, 16) der Warenbahn (11) erfolgt.

4. Verfahren nach einem der Ansprüche 1 bis 3,
**dadurch gekennzeichnet,**
daß die pyrometrische Abtastung zur Ermittlung des Feuchteprofils derart erfolgt, daß in einem Abtastvorgang ein komplettes Strahlungsbild des vorgegebenen Erfassungsbereichs (12) aufgenommen und daraus ein Feuchteprofil (20) abgeleitet wird.

5. Verfahren nach einem der Ansprüche 1 bis 4,
**dadurch gekennzeichnet,**
daß das in der zweiten Feuchtemessung erhaltene Feuchteprofil (20) mit dem in der ersten Feuchtemessung erhaltenen Absolutwert skaliert wird.

6. Verfahren nach einem der Ansprüche 1 bis 5,
**dadurch gekennzeichnet,**
daß die erste Feuchtemessung mittels Abtastung des elektrischen Widerstandes zwischen einer ersten Meßstelle (13) und einer zweiten Meßstelle (14) auf der Warenbahn (11) erfolgt.

7. Verfahren nach Anspruch 6,
**dadurch gekennzeichnet,**
daß die Abtastung zwischen einer ersten Flachseite (15) und der gegenüberliegenden Flachseite (16) der Warenbahn (11) erfolgt.

8. Verfahren nach Anspruch 6 oder 7,
**dadurch gekennzeichnet,**
daß die Abtastung des elektrischen Widerstandes zwischen einem oder mehreren Paaren (17) von Walzen (18, 19) erfolgt.

9. Vorrichtung zur Durchführung des Verfahrens nach einem der Ansprüche 1 bis 8, umfassend:
- eine Führungs- oder Transporteinrichtung (21), durch welche die Warenbahn (11) in gespanntem Zustand zu Zwecken einer Feuchteerfassung hindurchgeführt wird,
- eine erste Abtasteinrichtung (22), mit der ein Absolutwert des Feuchtegrades ermittelt wird,
- eine zweite Abtasteinrichtung (23) zur Erfassung eines Feuchteprofils insbesondere quer zur Warenbahn (11) über einen vorgegebenen Erfassungsbereich.

10. Vorrichtung nach Anspruch 9,
**dadurch gekennzeichnet,**
daß die erste Abtasteinrichtung (22) Rollen (18, 19) oder Walzen umfaßt und lokal den elektrischen Widerstand der feuchten Warenbahn ermittelt, und
daß die zweite Abtasteinrichtung ein Pyrometer (23) zur kontaktlosen Abtastung des Feuchteprofils (20) umfaßt.
